# EUROPÄISCHE PATENTSCHRIFT

(11) **EP 1 534 099 B1**
(45) Veröffentlichungstag und Bekanntmachung des Hinweises auf die Patenterteilung: **14.10.2009**
(21) Anmeldenummer: 03750421.4
(22) Anmeldetag: 21.08.2003
(51) Int. Cl.: A45D 29/00

(54) **VERFAHREN UND VORRICHTUNG ZUR VORBEREITUNG EINES FINGER- ODER FUSSNAGELS FÜR EINE BESCHICHTUNG, INSBESONDERE LACKIERUNG**
METHOD AND DEVICE FOR PREPARING A FINGERNAIL OR A TOE-NAIL FOR COATING, IN PARTICULAR WITH VARNISH
PROCEDE ET DISPOSITIF PERMETTANT DE PREPARER UN ONGLE D'UN DOIGT DE LA MAIN OU DU PIED POUR L'APPLICATION D'UN REVETEMENT, EN PARTICULIER D'UN VERNIS

(30) Priorität: 24.08.2002 DE 10238931
(43) Veröffentlichungstag der Anmeldung: 01.06.2005
(73) Patentinhaber: Viöl, Wolfgang, 37139 Adelebsen (DE)
(72) Erfinder: VIÖL, Wolfgang, 37139 Adelebsen (DE); VIÖL, Christian, 37139 Adelebsen (DE)
(74) Vertreter: Rehberg Hüppe + Partner
(86) Internationale Anmeldenummer: PCT/EP2003/009278
(87) Internationale Veröffentlichungsnummer: WO 2004/023927

(56) Entgegenhaltungen:
- EP-A- 0 523 961
- DE-C- 19 957 775
- FR-A- 2 415 439
- US-B1- 6 401 724

## Beschreibung

Die Erfindung bezieht sich auf ein Verfahren zur Vorbereitung eines Finger oder-Fußnagels für eine Beschichtung, insbesondere Lackierung, mit den Merkmalen des Oberbegriffs des unabhängigen Patentanspruch 1 und auf eine Vorrichtung zur Durch- führung eines solchen Verfahrens. Eine Vorbereitung eines Finger- oder Fußnagels für eine Beschichtung, insbesondere Lackierung, ist notwendig, um einerseits eine ausreichende Benetzung des Fingernagels ohne Einsatz größerer Mengen des Beschichtungsmaterials und andererseits eine halbwegs dauerhafte Beschichtung zu erreichen.

Es ist bekannt, Finger- oder Fußnägel für die Beschichtung, insbesondere die Lackierung, mit Lösungsmitteln zu reinigen und entfetten. Dennoch blättert ein anschließend aufgetragener Nagellack nach seiner Durchhärtung häufig nach relativ kurzer Zeit ab. Als Ergebnis müssen die Reste des Nagellacks entfernt und eine Neulackierung vorgenommen werden, um einen optisch befriedigenden Zustand des Finger- oder Fußnagels aufrecht zu erhalten.

Die EP 0 523 961 A1 beschreibt ein Verfahren zum Ablagern einer kosmetischen Farbzusammensetzung auf die Haut oder andere Teile des Körpers, wie beispielsweise Fingernägel. Die Zusammensetzung wird elektrostatisch aufgesprüht. Dazu wird an eine Auftragseinrichtung für die Zusammensetzung eine Hochspannung von einem Hochspannungsgenerator angelegt.

Der Erfindung liegt die Aufgabe zugrunde, ein neues Verfahren zur Vorbereitung eines Finger- oder Fußnagels für eine Beschichtung, insbesondere Lackierung, aufzuzeigen, nach dem es möglich ist, mit weniger Beschichtungsmittel, d. h. in der Regel Nagellack, eine dauerhaftere Beschichtung auszubilden. Weiterhin soll eine Vorrichtung zur Durchführung des neuen Verfahrens aufgezeigt werden.

Die Aufgabe wird durch ein Verfahren mit den Merkmalen des unabhängigen Patentanspruchs 1 und durch eine Vorrichtung mit den Merkmalen des Patentanspruchs 8 gelöst.

Grundsätzlich ist der Einsatz von so genannten Korona- oder Barriereentladungen bei Atmosphärendruck zur Behandlung von Kunststoffen, Keramiken u. dgl. zwecks Verbesserung deren Beschichtungseigenschaften seit längerem bekannt.

Aus der DE 199 57 775 C1 ist ein entsprechendes Verfahren zur Oberflächenmodifikation von Holz bekannt, bei dem das Holz selbst als Gegenelektrode für die Entladung dient.

Das neue Verfahren funktioniert im Wesentlichen analog dem bekannten Verfahren zur Behandlung von Holzoberflächen. Zur Auslösung der Gasentladung unter Atmosphärendruck über dem Nagel wird eine Hochspannung zwischen der Elektrode und dem Nagel angelegt.

In jedem Fall modifiziert die Gasentladung unter Atmosphärendruck die Oberfläche des Nagels so, dass die Oberflächenenergie stark ansteigt. Dies führt beispielsweise dazu, dass sich später aufgetragener Nagellack sehr gut auf dem Nagel verteilt und hervorragend an dem Nagel haftet. Wenn die gesamte Oberfläche des Nagels modifiziert wurde, kann beobachtet werden, dass sich ein lokal aufgetragener Nagellacktropfen vollständig über die Oberfläche des Nagels ausbreitet und diesen bis zum Rand hin abdeckt, ohne dass er dorthin verstrichen werden muss. Obwohl der Nagellack auf einem nach dem neuen Verfahren vorbehandelten Nagel deutlich besser anhaftet, kann er von diesem durch üblichen Nagellackentferner wieder entfernt werden. Die besonders gute Haftung des Nagellacks auf einem nach dem neuen Verfahren behandelten Nagel kann auch dazu genutzt werden, umweltfreundlichere Nagellacke, d. h. insbesondere Nagellacke mit weniger oder anderen Lösungsmitteln als bislang zu verwenden, die ohne die erfindungsgemäße Vorbehandlung zu keinen brauchbaren Lackierungsergebnissen führen. Die Vorteile der Erfindung sind auch nutzbar, um beispielsweise falsche Fingernägel oder andere Dinge an einen Nagel anzukleben und dabei eine bessere Verklebung zu erzielen, als dies bislang möglich war.

Obwohl bei der bevorzugten Ausführungsform die Gasentladung unter Atmosphärendruck über dem Nagel dadurch hervorgerufen wird, dass eine Hochspannung zwischen der Elektrode und dem Nagel angelegt wird, sind die dabei fließenden Ströme ungefährlich, wenn auf eine Begrenzung dieser Ströme geachtet wird, was dem Behandlungsergebnis des Nagels nicht abträglich sein muss.

Zum Anlegen der Hochspannung zwischen der Elektrode und dem Nagel kann die Haut des den Nagel tragenden Fingers mit Abstand zu dem Nagel elektrisch kontaktiert werden. Dies kann dazu dienen, den Nagel zu erden, insbesondere dann, wenn die Hochspannung gegenüber der Erde erzeugt wird. Wenn die Hochspannung jedoch zwischen zwei Hochspannungsausgängen erzeugt wird, sollte der eine mit der Elektrode und der andere mit dem jeweiligen Finger kontaktiert werden.

Die Gasentladung bei dem neuen Verfahren wird vorzugsweise in Luft ausgelöst, also in dem Gas, das die natürliche Umgebung des Nagels bildet.

Zur Begrenzung der über die Gasentladung fließenden Ströme ist die Gasentladung dielektrisch behindert. Das heißt, zwischen der Elektrode und dem Nagel wird eine dielektrische Schicht angeordnet. Eine gewisse dielektrische Behinderung ergibt sich durch die geringe Leitfähigkeit des Materials des Nagels selbst.

Bei einer dielektrisch behinderten Entladung ist es notwendig, dass die Hochspannung, welche zwischen dem Nagel und der Elektrode angelegt wird, eine Wechselhochspannung ist. Auch bei einer nicht dielektrisch behinderten Entladung kann die Hochspannung eine Wechselhochspannung sein. Bevorzugt ist eine Wechselhochspannung im Frequenzbereich von wenigen Hertz, weil diese besonders einfach, beispielsweise mit einem Piezo-Generator erzeugt werden kann.

Eine weitere Maßnahme zur Beschränkung der durch die Gasentladung fließenden Ströme besteht darin, dass die Hochspannung eine gepulste Hochspannung ist. Wenn gleichzeitig eine dielektrische Behinderung der Gasentladung vorgesehen ist, sollten die Pulse abwechselnd umgekehrte Polarisierungen aufweisen.

Vorzugsweise wird die Elektrode beim Hervorrufen der Gasentladung in einem Abstand von 1-5 mm zu dem Nagel angeordnet. Die notwendige Hochspannung zur Erzeugung der Gasentladung liegt dabei im kV-Bereich.

Als weiteres Mittel zum Begrenzen des durch die Gasentladung fließenden Stroms ist es sinnvoll, nur eine Gasentladung von kleinem Volumen auszulösen, d. h. nur ein einziges Entladungsfilament zwischen der Elektrode und dem Nagel auszubilden. Dieses Entladungsfilament kann dann durch Verschieben der Elektrode relativ zu dem Nagel benutzt werden, um die gesamte Oberfläche des Nagels für seine Beschichtung zu modifizieren. Dabei ist ein sehr feinfühliges lokales Arbeiten mit dem Entladungsfilament möglich.

In der Praxis stellt sich heraus, dass der Abstand zwischen der Elektrode und dem Nagel für ein gutes Resultat der Beschichtungsvorbereitung, nicht zu klein sein darf. Bevorzugt ist ein Abstand von einigen wenigen Millimetern.

Bei der Vorrichtung zur Durchführung des neuen Verfahrens kann ein mit der Elektrode verbundener Abstandhalter zur Definition eines Abstands zwischen der Elektrode und dem Nagel vorgesehen sein, und die Hochspannungsausgänge des Hochspannungsgenerators Können einerseits an die Elektrode und andererseits an einen Kontakt zur Kontaktierung des den Nagel tragenden Fingers oder der zugehörigen Hand in Abstand zu dem Nagel angeschlossen sein. Mit dem Abstandhalter wird ein definierter Abstand der Elektrode zu dem Nagel eingestellt. Die Elektrode wird entweder innerhalb des Abstandhalters bewegt, um die gesamte Oberfläche des Nagels abzuscannen, oder gemeinsam mit dem Abstandhalter.

Die Elektrode weist vorzugsweise eine dem Nagel zugewandte Elektrodenoberfläche mit einem Krümmungsradius < 1 mm auf. Dies sorgt auch bei begrenzten Hochspannungen für sehr große lokale Feldstärken. Hierdurch wird die Zündung der Gasentladung vereinfacht.

Der Abstandhalter der neuen Vorrichtung ist vorzugsweise aus einem elektrisch isolierenden Material ausgebildet, um Kurzschlüsse über den Abstandhalter zu vermeiden.

Wie bereits angesprochen, ist die Elektrode vorzugsweise über den Nagel hinweg beweglich. Gleichzeitig sollte die Haut durch den Kontakt, der mit dem anderen Hochspannungsausgang des Hochspannungsgenerators verbunden ist, durchgängig kontaktierbar sein.

An dieser Stelle ist anzumerken, dass die Gasentladung an der Oberfläche des mit ihr behandelten Nagels keine unangenehmen Reizungen oder Irritationen hervorruft. Die spürbaren Nervenreizungen sind deutlich geringer als diejenigen, die nach einer statischen Aufladung bei einem Kontakt mit einem metallischen Gegenstand erfahren werden. Dies lässt sich auf die relative Unempfindlichkeit von Finger- und Fußnägeln zurückführen. Der reine Stromfluss durch den Finger bis zu dem Kontakt wird als wenig störend empfunden, soweit er überhaupt wahrgenommen wird.

Eine besonders kostengünstig zur Verfügung stellbare Ausführungsform der neuen Vorrichtung weist als Hochspannungsgenerator einen Piezo-Generator auf. Piezo-Generatoren werden in Gasanzündern, Feuerzeugen u. dgl. in sehr großen Stückzahlen eingesetzt. So kann beispielsweise der Piezo-Generator eines Gasanzünders in der neuen Vorrichtung verwendet werden. So ist es möglich, die neue Vorrichtung auf bekannter Technik basierend kostengünstig herzustellen.

Die Erfindung wird im Folgenden anhand von zwei Ausführungsbeispielen näher erläutert und beschrieben. Dabei zeigt:
- Fig. 1: die prinzipielle Anordnung der neuen Vorrichtung bei der Durchführung des neuen Verfahrens in einer ersten Ausführungsvariante und
- Fig. 2: die prinzipielle Anordnung der neuen Vorrichtung bei der Durchführung des neuen Verfahrens in einer zweiten Variante mit dielektrischer Behinderung der Gasentladung.

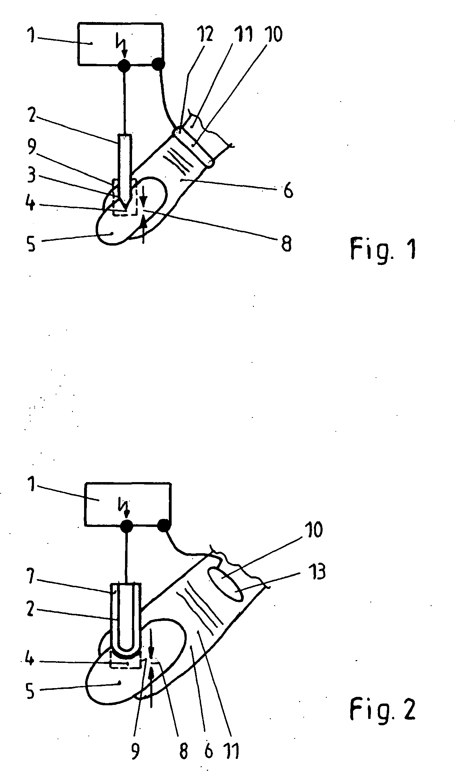

In **Fig. 1** ist ein Fingernagel 5 eines Fingers 6 gezeigt. Über dem Fingernagel 5 ist eine Elektrode 2 mit einer spitz zulaufenden Elektrodenspitze 3 angeordnet. Dabei hält die Elektrodenspitze 3 zu dem Nagel 5 einen Abstand 8 ein, der durch einen mit gestrichelter Linie angedeuteten Abstandhalter 9 vorgegeben wird. Die Elektrode 2 ist an einen von zwei Hochspannungsausgängen eines Hochspannungsgenerators 1 angeschlossen. Der andere Hochspannungsausgang ist mit einem Kontakt 10 verbunden, der an der Haut 11 des Fingers 6 mit Abstand zu dem Nagel 5 flächig anliegt. Der Kontakt 10 ist hier als Kontaktring 12 ausgebildet. Eine von dem Hochspannungsgenerator 1 hervorgerufene Hochspannung im kV-Bereich ruft eine Gasentladung 4 zwischen der Elektrodenspitze 3 und dem Nagel 5 hervor. Die Gasentladung 4 bildet sich in Form eines lokalisierten Entladungsfilaments aus und modifiziert die Oberfläche des Nagels 5 so, dass die Oberflächenenergie deutlich heraufgesetzt wird. Damit wird eine anschließende Beschichtung des Fingernagels 5, beispielsweise mit einem Nagellack, erleichtert, weil sich der Nagellack leichter über die gesamte Oberfläche des Nagels 5 verteilt. Außerdem haftet er hieran besser an. Um die gesamte Oberfläche des Nagels 5 entsprechend zu modifizieren, muss natürlich die Elektrodenspitze 3 mit dem von dieser ausgehenden Gasentladung 4 über die gesamte Oberfläche des Nagels 5 hinweg bewegt werden.

Die in **Fig. 2** illustrierte Ausführungsform der Erfindung unterscheidet sich von derjenigen in Fig. 1 dadurch, dass die Elektrode 2 durch ein Dielektrikum 7 abgeschirmt ist, so dass die Gasentladung 4 dielektrisch behindert ist. Auf diese Weise wird der Strom, welcher über den Finger 6 fließt, begrenzt. Er kann (auch bei der Ausführungsform gemäß Fig. 1) zudem dadurch klein gehalten werden, dass der Hochspannungsgenerator 1 einzelne, beabstandete Hochspannungspulse erzeugt. Unter diesen Randbedingungen können gefährliche Stromdichten durch den Finger 6 zuverlässig verhindert werden. Der Abstandhalter 9 kann aus demselben Material wie das Dielektrikum 7 ausgebildet sein. Er besteht vorzugsweise ohnehin aus nicht elektrisch leitendem Material. Der Kontakt 10 ist gemäß Fig. 2 kein Kontaktring, sondern ein begrenztes Kontaktstück 13. In jedem Fall bewirkt die Kontaktierung der Haut 11 des Fingers 6 auch im Abstand zu dem Fingernagel 5, dass der Fingernagel 5 als Gegenelektrode für die Gasentladung 4 über dem Nagel 5 wirkt. Die Gasentladung 4 erfolgt unter Atmosphärendruck und üblicherweise in Luft. Der Luft können aber, falls erwünscht, bestimmte Gase zugesetzt werden, die eine zusätzliche Modifikation der Oberfläche des Nagels 5 bewirken können.

Die Modifikation des Nagels 5 durch eines der in den Fig. 1 und 2 illustrierten Verfahren hält für einige Minuten an, so dass zunächst alle Finger einer Hand vorbehandelt und dann anschließend beispielsweise mit Nagellack beschichtet werden können. Es können auch zunächst alle zehn Finger oder alle zehn Fußnägel vorbehandelt werden, bevor die Lackierung vorgenommen wird.

### BEZUGSZEICHENLISTE

- 1: Hochspannungsgenerator
- 2: Elektrode
- 3: Elektrodenspitze
- 4: Gasentladung
- 5: Nagel
- 6: Finger
- 7: Dielektrikum
- 8: Abstand
- 9: Abstandhalter
- 10: Kontakt

- 11: Haut
- 12: Kontaktring
- 13: Kontaktstück

## Patentansprüche

1. Verfahren zur Vorbereitung eines Finger- oder Fußnagels (5) für eine Beschichtung, insbesondere Lackierung, mit dem Schritt: Auslösen einer Gasentladung (4) unter Atmosphärendruck über dem Nagel (5), **dadurch gekennzeichnet, dass** die Gasentladung (4) dielektrisch behindert wird.

2. Verfahren nach Anspruch 1, **dadurch gekennzeichnet, dass** eine Hochspannung zwischen einer Elektrode (2) und dem Nagel (5) angelegt wird.

3. Verfahren nach Anspruch 2, **dadurch gekennzeichnet, dass** zum Anlegen der Hochspannung zwischen der Elektrode (2) und dem Nagel (5) die Haut (11) des den Nagel (5) tragenden.Fingers (6) mit Abstand zu dem Nagel (5) elektrisch kontaktiert wird.

4. Verfahren nach Anspruch 2 oder 3, **dadurch gekennzeichnet, dass** die Gasentladung (4) in Luft ausgelöst wird.

5. Verfahren nach einem der Ansprüche 2 bis 4, **dadurch gekennzeichnet, dass** die Hochspannung eine Wechselhochspannung im Frequenzbereich von 1 bis 10 Hz ist.

6. Verfahren nach einem der Ansprüche 2 bis 5, **dadurch gekennzeichnet, dass** die Hochspannung eine gepulste Hochspannung ist.

7. Verfahren nach einem der Ansprüche 2 bis 6, **dadurch gekennzeichnet, dass** die Elektrode (2) in einem Abstand (8) von 1 bis 5 mm zu dem Nagel (5) angeordnet wird und dass die Hochspannung im kV-Bereich liegt.

8. Vorrichtung zur Durchführung des Verfahrens nach einem der Ansprüche 1 bis 7, wobei eine Elektrode (2) zur Anordnung über dem Nagel (5) vorgesehen ist und wobei ein Hochspannungsgenerator (1) vorgesehen ist, um eine Wechselhochspannung zu erzeugen und an die Elektrode (2) anzulegen, **dadurch gekennzeichnet, dass** die Elektrode (2) durch ein vor der Elektrode angeordnetes Dielektrikum (7) abgeschirmt ist.

9. Vorrichtung nach Anspruch 8, **dadurch gekennzeichnet, dass** der Hochspannungsgenerator (1) die Wechselhochspannung gegenüber der Erde erzeugt.

10. Vorrichtung nach Anspruch 8 oder 9, **dadurch gekennzeichnet, dass** die Wechselhochspannung eines gepulste Wechselhochspannung aus abwechselnden Pulsen umgekehrter Polarisierung ist.

11. Vorrichtung nach einem der Ansprüche 8 bis 10.
**dadurch gekennzeichnet, dass** ein mit der Elektrode (2) verbundener Abstandhalter (9) zur Definition eines Abstands (8) zwischen der Elektrode (2) und dem Nagel (5) vorgesehen ist, und **dass** die Hochspannungsausgänge des Hochspannungsgenerators (1) einerseits an die Elektrode (2) und andererseits an einen Kontakt (10) zur Kontaktierung des den Nagel (5) tragenden Fingers (6) oder der zugehörigen Hand in Abstand zu dem Nagel (5) angeschlossen sind.

12. Vorrichtung nach einem der Ansprüche 8 bis 11, **dadurch gekennzeichnet, dass** die Elektrode (2) eine dem Nagel (5) zugewandte Elektrodenoberfläche mit einem Krümmungsradius kleiner als 1 mm aufweist.

13. Vorrichtung nach Anspruch 11, **dadurch gekennzeichnet, dass** der Abstandhalter (9) aus einem elektrisch isolierenden Material ausgebildet ist.

14. Vorrichtung nach einem der Ansprüche 11 und 13, **dadurch gekennzeichnet, dass** die Elektrode (2) über den Nagel (5) beweglich ist, während die Haut (11) durch den Kontakt (10) durchgängig kontaktierbar ist.

15. Vorrichtung nach einem der Ansprüche 8 bis 14, **dadurch gekennzeichnet, dass** der Hochspannungsgenerator (1) ein Piezo-Generator ist.

## Claims

1. Method for pre-treating a finger-nail or toe nail (5) for a coating, particularly with varnish, comprising the step: igniting an gas discharge (4) under atmospheric pressure above the nail (5), **characterized in that** the gas discharge (4) is a dielectric barrier discharge.

2. Methods of claim 1, **characterized in that** a high voltage is applied between an electrode (2) and the nail (5).

3. Method of claim 2, **characterized in that** for applying the high voltage between the electrode (2) and the nail (5) the skin (11) of the finger (6) carrying the nail (5) is electrically contacted at a distance to the nail (5).

4. Method of claim 2 or 3, **characterized in that** the gas discharge (4) is ignited in air.

5. Method of any of the claims 2 to 4, **characterized in that** the high voltage is an alternating high voltage in the frequency range of 1 to 10 Hz.

6. Method of any of the claims 2 to 5, **characterized in that** the high voltage is a pulsed high voltage.

7. Method of any of the claims 2 to 6, **characterized in that** the electrode (2) is arranged at a distance (8) of 1 to 5 mm to the nail (5) and that the high voltage is in the kV-range.

8. Device for carrying out the method of any of the claims 2 to 7, wherein an electrode (2) is provided for being arranged above the nail (5) and wherein a high voltage generator (1) is provided to generate and apply an alternating high voltage to the electrode (2), **characterized in that** the electrode (2) is shielded by a dielectric (7) arranged in front of the electrode.

9. Device according to claim 9, **characterized in that** the high voltage generator (1) generates the alternating high voltage with respect to earth.

10. Device according to claim 9 or 10, **characterized in that** the alternating high voltage is a pulsed alternating high voltage made of alternating pulses of opposite polarity.

11. Device according to any of the claims 8 to 10, **characterized in that** a spacer (9) connected to the electrode (2) is provided for defining a distance (8) between the electrode (2) and the nail (5), and that the high voltage outputs of the high voltage generator (1) are connected to the electrode (2) at the one hand, and to an contact (10) for contacting the finger (6) carrying the nail (5) or the associated hand at a distance to the nail (5) on the other hand.

12. Device according to any of the claims 8 to 11, **characterized in that**, the electrode (2) comprises a electrode surface facing the nail (5) and having a radius of curvatures of less than 1 mm.

13. Device according to claim 11, **characterized in that** the spacer (9) is made of an electrically isolating material.

14. Device of any of the claims 11 and 13, **characterized in that** the electrode (2) is moveable over the nail (5), whereas the skin (11) is continuously contactable by the contact (10).

15. Device of any of the claims 8 to 14, **characterized in that**, the high voltage generator (1) is a piezo-generator.

## Revendications

1. Procédé de préparation d'un ongle de doigt ou d'orteil (5) pour application d'un revêtement, notamment d'un vernis, avec l'étape de déclenchement d'une décharge de gaz, **caractérisé en ce que** la décharge de gaz (4) est gênée diélectriquement.

2. Procédé selon la revendication 1, **caractérisé en ce qu'**une haute tension est appliquée entre une électrode (2) et l'ongle.

3. Procédé selon la revendication 2, **caractérisé en ce que** pour appliquer la haute tension entre l'électrode (2) et l'ongle (5) un contact est réalisé avec la peau (11) du doigt (6) portant l'ongle (5); à l'écart de l'ongle (5).

4. Procédé selon la revendication 2 ou 3, **caractérisé en ce que** la décharge de gaz (4) est diluée dans l'air.

5. Procédé selon l'une des revendications 2 à 4, **caractérisé en ce que** la haute tension est une tension alternative dans la plage de fréquence de 1 à 10 Hz.

6. Procédé selon l'une des revendications 2 à 5, **caractérisé en ce que** la haute tension est une haute tension pulsée.

7. Procédé selon l'une des revendications 2 à 6, **caractérisé en ce que** l'électrode (2) est disposée avec un écart (8) de 1 à 5 mm de l'ongle (5) et **en ce que** la haute tension se trouve dans la zone des kV.

8. Dispositif pour l'exécution du procédé selon l'une des revendications 1 à 7 dans lequel il est prévu une électrode (2) devant être disposée par dessus l'ongle (5) et dans lequel il est prévu un générateur de haute tension (1) pour générer une haute tension alternative et l'appliquer à l'électrode (2) **caractérisé en ce que** l'électrode (2) est blindée par un diélectrique (7) disposé devant l'électrode.

9. Dispositif selon la revendication 8, **caractérisé en ce que** le générateur de haute tension (1) produit la haute tension alternative par rapport à la terre.

10. Dispositif selon la revendication 8 ou 9, **caractérisé en ce que** la haute tension alternative est une haute tension alternative pulsée au moyen d'impulsions alternatives de polarités opposées.

11. Dispositif selon l'une des revendications 8 à 10, **caractérisé en ce qu'**il est prévu un écarteur (9) solidaire de l'électrode (2) pour définir un écart (8) entre l'électrode (2) et l'ongle (5) et **en ce que** les sorties haute tension du sont reliées d'une part à l'électrode (2) et d'autre part à un contact (10) pour établir le contact avec le doigt (6) portant l'ongle (5), ou avec la main correspondante, avec un écart par rapport à l'ongle (5).

12. Dispositif selon l'une des revendications 8 à 11, **caractérisé en ce que** l'électrode (2) présente une surface tournée vers l'ongle (5) avec un rayon de courbure inférieur à 1 mm.

13. Dispositif selon la revendication 11, **caractérisé en ce que** l'écarteur (9) est réalisé en une matière électriquement isolante.

14. Dispositif selon l'une des revendications 11 et 13, **caractérisé en ce que** l'électrode (2) est déplaçable au-dessus de l'ongle (5) tandis que la peau (11) peut être contactée de façon conductrice par le contact (10).

15. Dispositif selon l'une des revendications 8 à 14, **caractérisé en ce que** le générateur de haute tension (1) est un générateur piezo.
